**Europäisches Patentamt**

**European Patent Office**

**Office européen des brevets**

(19)

(11) Publication number : **0 514 208 A1**

(12)

# EUROPEAN PATENT APPLICATION

(21) Application number : **92304433.3**

(51) Int. Cl.⁵ : **A61K 39/295, A61K 47/12**

(22) Date of filing : **15.05.92**

(30) Priority : **17.05.91 US 701918**

(43) Date of publication of application :
**19.11.92 Bulletin 92/47**

(84) Designated Contracting States :
**AT BE CH DE DK ES FR GB GR IT LI LU NL PT SE**

(71) Applicant : **MERCK & CO. INC.**
**126, East Lincoln Avenue P.O. Box 2000**
**Rahway New Jersey 07065-0900 (US)**

(72) Inventor : **Provost, Philip J.**
**1680 Jacks Circle**
**Lansdale, PA 19446 (US)**
Inventor : **Nalin, David R.**
**100 Lucky Hill Road**
**West Chester, PA 19382 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Non-stinging measles-mump-rubella vaccine composition.**

(57) The composition of this invention comprises a non-stinging vaccine formulation of live attenuated viruses, including measles, mumps, or rubella, either alone or a combination thereof. The vaccine has a stabilizer component which can contain any of the stabilizers already described in the art, but specifically limited to more than about 0.005 and less than about 0.075 M phosphate, buffered at a pH between about 6.0 and 7.0. The composition is superior to known vaccine compositions in that it has reduced stinging properties upon administration.

EP 0 514 208 A1

## BACKGROUND OF THE INVENTION

Patient tolerance of vaccine administration may be a significant factor in obtaining compliance with recommendations by the Center for Disease Control. Currently available live attenuated virus vaccine compositions have been reported to cause stinging. For example, the trivalent measles, mumps, and rubella virus containing M-M-R®$_{II}$ vaccine has been reported to cause stinging upon administration (PDR 1990, p. 1402). This stinging response has been attributed to the slightly acidic pH of the vaccine stabilizer. Upon performing the definitive experiments, the instant invention was made wherein a composition of lowered phosphate concentration has reduced stinging, while retaining viral stability.

Live attenuated viruses and methods for preparing these viruses, useful for the preparation of the composition of this invention, are known in the art. For example, U.S. Patent 3,555,149 discloses a mumps virus and a method for its preparation. Enders, J. F. et al., [Proc. Soc. Exp. Biol. Med. 86, 227 (1954)] reported on the Edmonston strain of measles virus, while Rubella strains are known such as RA27/3 strain Wistar. In general, viruses of attenuated virulence may be prepared by passaging virulent viral isolates in an in vitro cell culture system, which technique is well known in the art.

Stabilizers of attenuated live viral vaccines are known in the art and are used to retain viability of cold-stored or lyophilized vaccine preparations. Known low phosphate stabilizers include SPGA, which contains 0.218M sucrose, 0.0038M monopotassium phosphate, 0.0072M dipotassium phosphate, 0.0049M monosodium glutamate, and 1% albumin. About 0.076M phosphte buffer was added to prior art formulations of trivalent MMR®.

Although virus stabilizers of low phosphate are known in the art, current compositions of trivalent measles, mumps and rubella for example, contains about 0.075 M phosphate or more. It was not recognized that a level of phosphate above about 0.075 M creates a stinging sensation upon administration of the composition. By carefully experimenting with placebo compositions, wherein a removal of one component at a time was matched with recipient responses in the clinic, a new composition was devised which has reduced stinging properties. Upon testing a complete vaccine, it was discovered that viral viability can be maintained while at the same time providing a vaccine that has reduced stinging properties.

## SUMMARY OF THE INVENTION

This invention is a novel composition comprising a live attenuated measles, mumps, rubella, polio, hepatitis, influenza or other virus, or a combination thereof, together with a vaccine stabilizer, having a limited amount of phosphate, being sufficient buffered phosphate between about 0.005 and less than about 0.075 moles per liter, to maintain the pH between about 6.0 and 7.0, but not so much that the composition stings upon administration.

## DETAILED DESCRIPTION OF THE INVENTION

The novel composition of this invention comprises any live attenuated virus in a stabilizer as described below. The virus is preferably an attenuated live measles, mumps, rubella, hepatitis A, influenza, rotavirus, or poliovirus prepared according to methods known in the art. In a preferred embodiment, the composition comprises, in addition to a stabilizer, a mixture of between two and four of said attenuated live viruses.

The composition has sufficient phosphate buffer to maintain the pH between about 6.0 and 7.0 but not so much that stinging occurs upon administration of the novel vaccine composition. An appropriate range of phosphate buffer for this purpose is more than about 0.005 and less than about 0.075 M phosphate, buffered at a pH between about 6.0 and 7.0. In a preferred embodiment of the invention, the composition contains about 0.0075 M of phosphate and has a pH of between 6 and 7. The composition also contains other acceptable components known in the art to stabilize the live virus components of the vaccine as well as the attenuated virus(es).

The measles virus may be an attenuated line of virus derived from Ender's attenuated Edmonston strain. The virus may be further attenuated by multiple passage in cell cultures of chick embryo at low temperature. About 1,000 or more TCID$_{50}$ (tissue culture infectious doses in terms of the assigned titer of the U.S. Reference Virus) should be included per dose in the disclosed stabilizer. Upon harvesting the measles virus, it is preferably stored frozen in a suitable liquid medium.

The mumps virus is preferably the Jeryl Lynn strain of mumps virus, adapted to and propagated in cell cultures of chick embryo. Approximately 20,000 TCID$_{50}$ of the virus should be included per dose in the disclosed stabilizer. Upon harvesting the mumps virus, it is preferably frozen in a suitable liquid medium. One medium for frozen storage of mumps virus is solution A, as described above.

The rubella virus is, for example, the RA 27/3 strain of live attenuated rubella virus grown in human diploid cell (WI-38 for example) culture according to methods known in the art. About 1,000 TCID$_{50}$ should be included

per dose in the disclosed stabilizer. Upon harvesting the rubella virus, it is preferably frozen in a suitable liquid medium.

In each case, the virus is harvested from culture at a concentration sufficient to allow dilution upon final formulation of the monovalent or multivalent vaccine. The virus preparation may contain residual culture medium components which, when added to the virus stabilizing components, form the novel composition of this invention. The cell culture medium or virus storage solutions such as used above or physiologic saline that is carried over in the preparation of this composition contributes minor components to the composition such as human albumin, phenol red, sodium bicarbonate, and culture medium such as medium 199 [Morgan et al., Proc. Soc. Exp. Biol. & Med. 73: 1-8 (1950)]; Basal Medium Eagle, [Eagle, Science 122:501-504 (1955); In Vitro 6, NO. 2 (1970)]; Dulbecco's Modified Eagle's Medium [Dulbecco et al., Virology 8:396 (1959); Smith et al., J. Virol. 12:185-196 (1960); In Vitro 6 No. 2 (1970)]; Minimal Essential Medium (Eagle) [Science 130:432 (1959)]; or RPMI Media [Moore et al., In Vitro 6 No.2 (1970)].

According to one embodiment of the invention, a virus composition comprises up to 25% by volume of virus. For example, the 25% volume may be comprised of measles (10%), mumps (10%), and rubella (5%). About 7.5% by volume is 0.1 M phosphate buffer, and the remaining 67.5 % of the volume is a stabilizer solution having less than 0.075M phosphate.

In any given embodiment where phosphate is added, any combination of water and different phosphate stock solutions which give a final phosphate concentration between 0.005 and 0.075M are acceptable. Thus, rather than adding, for example, 6.75% distilled water and 0.75% 1M phosphate, addition of 7.5% of 0.1M phosphate would give the same effective final concentration of phosphate, 0.0075 Molar.

In each case, the composition is prepared by mixing the appropriate volume ratios of components. The complete composition may then be frozen or lyophilized. The preferred method for storage of the composition is in the lyophilized state. The lyophilized preparation is simply reconstituted with water immediately prior to administration.

Vaccine compositions of this invention have been prepared and found to be efficacious in retaining the stability of the individual viruses. The compositions have minimal stinging properties upon administration of the vaccine due to the low phosphate buffer concentration, and the vaccine was efficacious in the prevention of infection by virulent strains of the virus when the attenuated counterpart was included in the vaccine composition.

The following examples are provided to extend the disclosure of the instant invention without limiting the invention to the specifics of the examples.

EXAMPLE 1

PREPARATION OF A TRIVALENT, LOW PHOSPHATE MEASLES, MUMPS, RUBELLA VACCINE:

A trivalent measles, mumps, rubella vaccine, M-M-R®$_{II}$, was compared with a low phosphate vaccine formulation, M-M-R®II$_{LP-1}$, for injection-site reactions following administration. The two vaccines were prepared as follows:

| SOLUTION | STANDARD M-M-R®II | LOW-PO$_4$ M-M-R®II$_{LP-1}$ |
|---|---|---|
| Low phosphate stabilizer | 67.5% | 67.5 % |
| Rubella solution | 5 % | 5 % |
| measles solution | 10 % | 10 % |
| Mumps solution | 10 % | 10 % |
| 1 M phosphate | 7.5% | 0.75% |
| Distilled water | 0 % | 6.75% |

The viral stocks used in the preparation of these compositions were prepared so that the final preparation contained at least 1000 TCID$_{50}$ of measles and rubella viruses per dose, and at least 20,000 TCID$_{50}$ of mumps virus per dose. The "standard" MMRII contained about 0.075 moles per liter of phosphate and Low Phosphate MMRII contained about 0.0075 moles per liter phosphate. The standard composition stung upon administration while the low phosphate composition did not.

Testing in human volunteers revealed reduced stinging of the low phosphate vaccine as compared with the standard composition. Analysis of the individual viruses in the high and low phosphate compositions revealed about equal viral stability in either composition.

EXAMPLE 2

PREPARATION OF A TRIVALENT, LOW PHOSPHATE MEASLES, MUMPS, RUBELLA VACCINE:

A trivalent measles, mumps, rubella vaccine with low phosphate, M-M-R®II$_{LP-2}$, may be prepared by replacing the water fraction in the M-M-R®II$_{LP-1}$ formulation of Example 1 with stabilizing components, and compared with the standard M-M-R®II:

| SOLUTION | STANDARD M-M-R®II | LOW-PO$_4$ M-M-R®II$_{LP-2}$ |
|---|---|---|
| Low Phosphate Stabilizer | 67.5% | 74.25% |
| Rubella solution | 5 % | 5 % |
| measles solution | 10 % | 10 % |
| Mumps solution | 10 % | 10 % |
| 1 M phosphate | 7.5% | 0.75% |

The viral stocks used in the preparation of these composition are prepared so that the final preparation contains at least 1000 TCID$_{50}$ of measles and rubella viruses per dose, and at least 20,000 TCID$_{50}$ of mumps virus per dose.

**Claims**

1. A vaccine composition comprising an attenuated live measles, mumps, and rubella; a stabilizer; and sufficient buffered phosphate, between about 0.005 and less than about 0.075 moles per liter, to maintain the pH between about 6.0 and 7.0, but not so much that the composition stings upon administration.

2. The composition of Claim 1 with a final phosphate concentration of about 0.0075 moles per liter.

3. The composition of Claim 2 comprising a dose of at least 1000 TCID$_{50}$ of measles and rubella viruses, and at least 20,000 TCID$_{50}$ of mumps.

4. The composition of Claim 2 in which rubella is 5%, measles is 10% and mumps is 10% of the composition.

5. A lyophilized form of the composition of any of Claims 1-4.

6. The use of the composition of Claim 1 for the manufacture of a medicament for vaccinating a human to prevent disease caused by one or more of measles, mumps, or rubella, virus infections, such that minimal stinging at the site of administration occurs.

**European Patent Office**

# EUROPEAN SEARCH REPORT

Application Number

EP 92 30 4433

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 252 059 (SMITH KLINE-RIT)<br>* page 3, line 1 - line 21 *<br>* page 5; example 4 *<br>--- | 1-6 | A61K39/295<br>A61K47/12 |
| A | EP-A-0 065 905 (INSTITUT PASTEUR)<br>* page 4, line 14 - page 6, line 30 *<br>--- | 1-6 | |
| A | EP-A-0 028 563 (MERCK & CO.INC.)<br>* the whole document *<br>--- | 1-6 | |
| A | BUNDESVERBAND DER PHARMAZEUTISCHEN INDUSTRIE E.V. 'rote liste'<br>1990 , EDITIO CANTOR , FRANKFURT<br>* abstract no. 74101* <br>----- | 1-6 | |
| | | | **TECHNICAL FIELDS SEARCHED (Int. Cl.5)**<br><br>A61K |

The present search report has been drawn up for all claims

| Place of search | Date of completion of the search | Examiner |
|---|---|---|
| THE HAGUE | 27 AUGUST 1992 | FERNANDEZ Y BRA F. |

EPO FORM 1503 03.82 (P0401)